Europäisches Patentamt

European Patent Office

Office européen des brevets

(19)

(11) Publication number: **0 145 467**
**B1**

# (12) EUROPEAN PATENT SPECIFICATION

(45) Date of publication of patent specification: **22.02.89**

(21) Application number: **84308536.6**

(22) Date of filing: **07.12.84**

(51) Int. Cl.⁴: **C 07 C 51/25, C 07 C 57/04, C 07 C 57/08** // B01J23/44

(54) Catalytic oxidation of olefins to alpha, beta-unsaturated carboxylic acids.

(30) Priority: **07.12.83 US 559057**
**29.10.84 US 664563**

(43) Date of publication of application:
**19.06.85 Bulletin 85/25**

(45) Publication of the grant of the patent:
**22.02.89 Bulletin 89/08**

(84) Designated Contracting States:
**BE DE FR GB IT NL**

(56) References cited:
**GB-A-1 361 811**
**GB-A-1 535 584**
**US-A-3 804 779**

The file contains technical information
submitted after the application was filed and
not included in this specification

(73) Proprietor: **Sun Refining and Marketing**
**Company**
**1801 Market Street**
**Philadelphia, PA 19103-1699 (US)**

(72) Inventor: **Lyons, James E.**
**211 Cooper Drive**
**Wallingford PA 19086 (US)**
Inventor: **Suld, George**
**201 W. Springfield Road**
**Springfield, PA 19064 (US)**

(74) Representative: **Lewin, John Harvey et al**
**ELKINGTON AND FIFE Beacon House 113**
**Kingsway**
**London WC2B 6PP (GB)**

Courier Press, Leamington Spa, England.

## Description

Background of the invention
Field of the invention

This invention relates to a process for the oxidation of olefins to α,β-unsaturated carboxylic acids. More particularly, this process is directed to the improvement of oxidizing propylene in one step to form acrylic acid at high selectivities and yields under moderate reaction conditions by the use of a novel olefin-activated palladium catalyst system. In a like manner, isobutylene and butene-1 may be oxidized respectively to methacrylic acid and crotonic acid.

This invention further relates to said novel activated palladium catalysts *per se* and methods of preparing the same.

Description of the prior art

The oxidation of propylene to acrylic acid in one step employing a palladium metal catalyst supported on carbon black is described in U.S. Patent 3,624,147. However, this process is characterized by yields of 60% or less, based on the amount of propylene converted, operating temperatures generally in excess of 90°C, and elevated pressures. Moreover, substantial amounts of $CO_2$ are reported as undesired by-products, as well as low reaction rates.

A similar process is reported in *J. Catal.*, 173 (1972) by Seiyama et al., in which palladium black and palladium-activated charcoal were employed for converting propylene to acrylic acid. However, only a stoichiometric, non-catalytic conversion, based on the palladium metal, is taught, thus providing an even less effective method than in the above U.S. Patent.

In addition, several patents describe conventional methods for the preparation of supported palladium metal catalysts by the reduction of, for example, palladium salts using such reducing agents as hydrogen, lower alcohols, hydrazine, or various olefins. See, for example, U.S. Patents 3,275,680 to Holzrichter, or 4,435,598 to Hinnenkamp which teach reducing palladium salts with hydrogen or hydrazine. U.S. Patent 4,016,200 to Onoda likewise teaches that a palladium compound can be reduced to palladium metal, using formalin, hydrazine, hydrogen, methanol or olefins such as ethylene, propylene, or butene as reducing agents. Similarly, U.S. Patent 3,970,713 to Scharfe teaches the reduction of palladium and other metal salts to a metal catalyst, again using hydrogen, alcohols, olefins, etc. as reducing agents. However, none of these references teaches the preparation of a highly activated species of palladium metal which has been activated with an olefin under unique time and temperature conditions, or that such a catalyst is surprisingly effective in a process for the oxidation of olefins to α,β-unsaturated acids under more moderate operating conditions than have heretofore been possible. Finally, F. R. Hartley, "The Chemistry of Platinum and Palladium", Wiley and Sons, pp. 386—390, and 412—417 (1973) disclose the formation of a complex of ethylene with a palladium chloride to provide a palladium$^{+2}$ metal catalyst. However, as will be described below, the use of ethylene, or chlorides, and the formation of palladium$^{+2}$ metal catalysts have been found to deactivate the catalyst of this invention for purposes of forming the desired products claimed herein.

Therefore, it is an object of this invention to provide an improved process for converting olefins such as propylene to α,β-unsaturated carboxylic acids such as acrylic acid in one step at high yields and selectivities as contrasted with reported prior art methods.

It is a further object of this invention to oxidize isobutylene to methacrylic acid and butene-1 to crotonic acid in the same manner.

It is yet a further object of this invention to provide a novel palladium catalyst useful in achieving the above ends, as well as a process for preparing said catalyst.

Other objects of this invention will be evident from the description and examples set forth hereinbelow.

Summary of the invention

In accordance with the present invention it has now been found that olefins such as propylene can be oxidized with air or oxygen in a single step in the liquid phase and under mild reaction conditions to form α,β-unsaturated carboxylic acids such as acrylic acid in high yield and at high selectivities when there is employed an activated palladium metal catalyst supported on carbon or alumina, wherein the supported palladium metal catalyst has first been activated with an olefin, preferably the olefin corresponding to the one to be oxidized, prior to said oxidation under conditions described in detail below. By this unique expedient, catalysts which were otherwise inactive at temperatures below 60°C are now not only active at much lower temperatures but also they provide molar selectivities to acrylic acid approaching at least 90%, thus virtually eliminating the formation of undesired $CO_2$.

This same catalyst system is likewise effective in oxidizing isobutylene to methacrylic acid, and butene-1 to crotonic acid.

Thus, olefins having from three to about six carbon atoms may be oxidized by the process of this invention.

## EP 0 145 467 B1

Description of the preferred embodiments and catalyst preparation

The general method of oxidizing propylene to acrylic acid is adequately described in the prior art and need not be described herein in detail. Suffice it to say that utilizing the catalyst of this invention, prepared by the novel methods discussed in detial below, the oxidation reaction of propylene to acrylic acid can then be uniquely carried out at temperatures in the range of from about 25 to 125°C, and at pressures of 1 to 100 atm. Preferably, temperatures of from 25 to 85°C, and pressures of from 1 to 10 atm may be employed, as contrasted with the much more rigorous conditions employed in U.S. 2,624,147. Moreover, as a consequence of this new catalyst, rates, selectivities, and thus yields, are significantly increased, as shown in the examples below.

In one preferred embodiment of this process, in order to increase the reaction rate and at the same time reduce the reactor volume, it has been found to be advantageous that the reaction be carried out in a trickle bed reactor in which the liquid reaction medium is allowed to pass downward over a fixed catalyst bed and the acrylic acid product recovered at the bottom. Alternatively, the oxidation reaction can be carried out using an ebulating bed of catalyst while circulating gases and solvent.

The starting material from which the novel catalyst of this invention is prepared may be any finely divided palladium in the metallic state, on a support such as carbon or, less preferred, alumina, as for example a commercially available 5%, 10%, or 20% palladium on carbon available from standard catalyst manufacturers such as Engelhard Industries or Johnson Mathey, Inc. By the terms "palladium metal catalyst" or "palladium in the metallic state" is meant those palladium catalysts which have been prepared from their salts by known reduction means either commercially or as shown, for example, by Scharfe et al, U.S. Patent 3,970,713, or Holzrichter et al, U.S. Patent 3,275,680, but which have subsequently been exposed to the atmosphere to normal process procedures. While applicants do not wish to be bound by any particular theories, it is believed that in the normal course of handling and using the reduced catalysts of the prior art subsequent to reduction of the palladium, a certain proportion of the palladium surface species, by virtue of exposure to the atmosphere, becomes oxidized. It is this air-exposed palladium catalyst which is now being employed as the starting material in the preparation of applicants' novel olefin-activated catalyst. (By "surface species", as recognized by those skilled in the catalyst art, is meant any species of palladium found at the surface of the catalyst per se.)

Again, while applicants do not wish to be bound by any particular theory, it is believed that when this partly oxidized palladium surface, as described above, is contacted with propylene in accordance with applicants' invention, it is first converted to highly active palladium metal sites having zero valence, and it is with these sites that the propylene then forms the novel surface-active species which is the activated catalyst of this invention.

As evidence that the commercially-reduced palladium, for example, has been reoxidized under normal handling and exposure to air, it has been found that in the course of preparing the novel activated catalyst of this invention, starting, e.g., with a commercially reduced palladium metal catalyst, under oxygen-free conditions as described below, two parts propylene employed in activating the catalyst result in the formation of one part acetone and one part active catalyst species.

In preparing the novel activated oxidation catalyst of this invention by treating a carbon- or alumina-supported palladium metal catalyst as defined above with propylene or like olefins, it is essential that this activation treatment be carried out at temperatures of at least about 60°C, up to 150°C, preferably about 65 to 95°C, for a period of at least about 10 minutes to about 120 minutes, preferably at least about 30 to 60 minutes, under oxygen-free conditions as described below. This is generally carried out at pressures of at least about 1 atmosphere, up to about 100 atmospheres of propylene, although about 2—20 atmospheres is preferred. When these catalysts are thus activated, palladium-on-carbon, for example, which was otherwise far less reactive at temperatures below about 60°C for purposes of oxidizing propylene is now surprisingly active at temperatures of about 25°C or above. Moreover, as aforestated, both the rates and selectivities to acrylic acid are significantly improved by this treatment. Thus, by the term "activated palladium metal catalyst" is meant, for purposes of this invention, a catalyst prepared in accordance with the above method, and which can oxidize propylene to acrylic acid more rapidly and at lower temperatures than known supported palladium catalysts.

During the preparation of the catalyst, as stated above, it is necessary for purposes of deriving maximum activity from the catalyst that the activation be carried out in the substantial absence of oxygen, and preferably under essentially oxygen-free conditions. While the presence of small amounts of oxygen, to an extent which can be readily determined by those skilled in the art, can still result in a catalyst which performs under somewhat more mild conditions than the commercial catalysts described above, the full benefits of the present invention are derived from activating the catalyst under conditions which are as oxygen-free as can be obtained, at least within the standards of commercial feasibility.

These oxygen-free conditions can be achieved by known means, for example by using deaerated water or solvent, and pure olefin gas, during the activation of the catalyst. Deaeration can be readily achieved by placing the liquid under vacuum until it boils, or by bubbling the desired olefin through the liquid for a period of time until no more oxygen is displaced. The pure olefin can be obtained commercially in various grades such as chemical purity grade, research purity grade, or polymer grade, the latter two being preferred because of their higher purity of over about 99.7%. (The latter two are available, for example from Matheson, Division of Searle Medical Products, and Sun Co., respectively).

3

Once applicants' catalyst is formed, it is preferable that at least a slight excess of olefin be present at all times to prevent any deactivation, and that desirably during the oxidation step, oxygen in the reactor be maintained in no greater than the stoichiometric amounts needed for the oxidation of the olefin to acrylic acid. It will also be understood that in preparing the catalyst of this invention, the presence of those metals or metal salts which might poison or alter the catalyst should be avoided, for example iron, manganese, copper and rhodium salts; chlorides, benzoquinone, the oxidized form of heteropoly acids, as well as any other agents which would oxidize palladium to palladium$^{+2}$. Other such deleterious materials can be routinely determined by those skilled in the art. For example, in addition, it has been found that such materials as amines, hydrazine, and ethylene should be avoided as deleterious when preparing and using the catalyst of this invention. Moreover, it has been found that attempts to use hydrogen to prepare this catalyst may result in explosions when the catalyst is exposed to $O_2$-propylene mixtures, and should also be avoided.

While the catalyst of the invention may be prepared separately and maintained in an active state if kept in an oxygen-free atmosphere, more conveniently the preparation is carried out in the same reactor used for the propylene oxidation. This may conveniently be achieved, for example by adding a commercially available finely divided palladium on activated carbon to an aqueous medium in a sealed reactor, flushing the system with propylene gas, and then heating the mixture under propylene pressure until the desired temperature for preparation of the catalyst is reached, at which time the mixture is stirred for at least 30 minutes at that temperature, again, in the absence of oxygen, and desirably in the presence of a slight excess of olefin.

After the preparation of the catalyst, the propylene may be replaced by a mixture of propylene and oxygen, desirably with oxygen being present in approximately stoichiometric amounts to avoid deactivation of the catalyst, and the oxidation reaction carried out at pressures of from about 1 to 10 atmospheres. The pressure may be maintained by the further addition of the gas mixture from time to time until the desired propylene conversion is achieved. Air may be used in place of oxygen, in which case the amount of propylene must be adjusted proportionately.

While the activating agent for the catalyst is preferably propylene, if desired there may instead by employed other light olefins having an allylic hydrogen and containing from 3—6 carbon atoms, preferably those corresponding to the olefins to be oxidized. Most preferred, in addition to propylene, are butene-1, butene-2 or isobutylene.

The olefin-activated catalyst maintains its activity over long periods of time as long as at least small amounts of an acceptable olefin are present. Thus, it has been found beneficial to run the reaction by constantly sparging the propylene/oxygen or air reaction mixture through the aqueous solution. In this way, the propylene is kept in excess and the catalyst remains highly active, thereby maintaining high selectivities and other advantages noted above.

When carrying out the oxidation in a batch-wise manner the ratio of catalyst to reaction medium is desirably in the range of about 0.05—5.0 gram atoms of palladium per liter of reactant, and preferably about 0.1—1.0 gram atoms. In a continuous process utilizing, e.g., a fixed bed reactor, the reaction can be conducted effectively by varying the volume of reactants and contact time with the catalyst in a generally known manner to achieve the high yields and selectivities disclosed herein.

The following examples are by way illustration of the invention.

Examples 1—9

In the following examples, 1—9, a number of reactions were run in accordance with the following general procedure.

One gram of 10% palladium metal on carbon (Engelhard Industries) was added to an 85 ml Fisher-Porter aerosol tube. Then 30 ml of deaerated distilled water was added and the Fisher-Porter tube was fitted to a pressure manifold. The mixture was flushed to 50 psi three times with pure propylene gas (research purity grade). It was then heated with stirring under 50 psi of this pure propylene until it reached the desired activation temperature where the mixture was stirred for 30 minutes. The stirred mixture was then brought to the desired reaction temperature and the propylene was replaced with a gas mixture having the composition: 60% $O_2$/40% pure $C_3H_6$ to a total pressure of 100 psig. Reaction proceeded immediately in most cases and the pressure dropped. When the total pressure reached 80 psig the $O_2/C_3H_6$ gas mixture was admitted to bring the total pressure to 100 psig. This was repeated as often as necessary during the course of the run. After the determined reaction time the mixture was cooled, the gas captured and analyzed and the mixture filtered. The catalyst was washed with both organic and aqueous solutions to remove small amounts of acrylic acid held on the surface. The filtrates were analyzed by standardized GC to determine the product composition.

As shown in Table 1 below, the conditions for preparing the catalyst and for oxidizing the propylene were varied from run to run to better illustrate the scope of the invention. Minor amounts of by-products such as acetone, acrolein, acetic acid, $CO_2$ and the like are also reported.

It can be seen from the results in Table I that employing a temperature of 65 or 80°C generates a catalyst which is active at 40 or 50°C. Similar treatment at 40 or 50°C gives a catalyst which is inactive at these temperatures. The catalyst generated at 80°C is also more selective than that generated at 65°C for oxidations at lower temperatures. Reactions carried out at 65°C are more rapid and selective than those done at 50°C with comparable activation temperatures. Catalysts treated at 80°C are active for oxidations at 30°C, but both rate and selectivity to acrylic acid are lower than at 65°C.

By contrast, Examples 7—9 illustrate that when a commercial catalyst is not subjected to the activation treatment described above, the yields and selectivities are negligible.

TABLE I

Effect of activation temperature on rate and selectivity of catalytic propylene oxidations

| Catalyst[a] | Prep temp.,°C | Reaction temp.,°C | Reaction time, hrs | Gas up-take, psi | Liquid analysis GMS/liter wt.% | | | | Acrylic acid production | | | | |
|---|---|---|---|---|---|---|---|---|---|---|---|---|---|
| | | | | | Acet-aldehyde | Acetone | Acrolein | Acetic acid | Acrylic acid | CO₂ mmoles | Yield g/l | Sel. % | Run rate psi/hr. |
| 1. 10% Pd-C, | 80 | 65 | 4 | 168 | 0.72 2.06 | 1.18 3.38 | 0.34 0.97 | 1.64 4.70 | 31.04 88.9 | 2.3 | 31.0 | 88.9 | 42.0 |
| 2. 10% Pd-C, | 65 | 65 | 4 | 165 | 0.46 1.51 | 1.43 4.70 | 0.21 0.70 | 1.12 3.68 | 27.2 89.4 | 2.8 | 27.2 | 89.4 | 41.3 |
| 3. 10% Pd-C, | 80 | 50 | 4 | 82 | 0.31 1.39 | 1.98 8.90 | 0.86 3.87 | 0.61 2.74 | 18.5 83.1 | 0.7 | 18.5 | 83.1 | 20.5 |
| 4. 10% Pd-C, | 68 | 50 | 4 | 82 | 0.20 1.80 | 2.13 19.14 | 0.53 4.76 | 2.57 23.09 | 5.7 51.21 | 0.8 | 5.7 | 51.2 | 20.5 |
| 5. 10% Pd-C, | 52 | 50 | 4 | 9 | — | 0.45 42.86 | — | 0.60 57.14 | — | 0.1 | — | — | 2.3 |
| 6. 10% Pd-C, | 80 | 40 | 7 | 71 | 0.15 0.63 | 0.75 3.16 | 0.18 0.76 | 2.81 11.82 | 19.88 83.63 | 0.4 | 19.9 | 83.6 | 10.1 |
| 7. 10% Pd-C, | 40 | 40 | 4 | 0 | — | — | — | — | — | — | — | — | — |
| 8. 10% Pd-C, | b | 65 | 4 | 9 | — | <1 | — | <1 | — | 0.4 | | | |
| 9. 10% Pd-C, | b | 65 | 4 | 10 | — | <1 | — | <1 | — | 0.25 | | | |

a) Engelhard Industries

b No activation period. Reaction mix was heated quickly to 65°C under a 60/40 $O_2/C_3$ mix and reaction run at 65°C.

The following examples, 10—12, illustrate the oxidation of isobutylene and butene-1, using the procedures of the foregoing examples.

Example 10

When the reaction was run in accordance with the procedures of Example 2 except that isobutylene was substituted for propylene, methacrylic acid was produced as a major product in high yield.

Example 11

When the reaction was run in accordance with the procedures of Example 4, except that isobutylene was substituted for propylene, methacrylic acid was produced as a major product in high yield.

Example 12

When the reaction was run in accordance with the procedures of Example 2, except that butene-1 was substituted for propylene, crotonic acid was produced as a major product in high yield.

Example 13

When 10% palladium on carbon was activated with propylene under conditions described in Example 6, and propylene oxidized in a similar manner, except that the reaction temperature was 30°C, acrylic acid was formed as a major reaction product.

**Claims**

1. A process for the preparation of α,β-unsaturated carboxylic acids which comprises contacting a supported palladium metal catalyst with a $C_3$—$C_6$ olefin admixed with air or oxygen, thereby oxidizing said olefin to the corresponding carboxylic acid, characterised in that the catalyst is first activated by contacting it with said $C_3$—$C_6$ olefin in an aqueous medium at a temperature of at least about 60°C for at least about 10 minutes in the substantial absence of oxygen.

2. A process for the preparation of α,β-unsaturated carboxylic acids which comprises contacting a supported palladium metal catalyst with a $C_3$—$C_6$ olefin admixed with air or oxygen, thereby oxidizing said olefin to the corresponding carboxylic acid, characterised in that the catalyst is first activated by contacting it with a different $C_3$—$C_6$ olefin in an aqueous medium at a temperature of at least about 60°C for at least about 10 minutes in the substantial absence of oxygen.

3. A process for the preparation of α,β-unsaturated carboxylic acid which comprises oxidizing a $C_3$—$C_6$ olefin admixed with air or oxygen in the presence of a palladium metal catalyst in a liquid medium, characterised in that said catalyst has been activated by contacting a supported palladium metal catalyst in said liquid medium with a different $C_3$—$C_6$ olefin at a temperature of at least about 60°C for at least about 10 minutes in the substantial absence of oxygen.

4. The process of any of Claims 1 to 3, wherein the catalyst is activated in the essential absence of oxygen.

5. The process of any of Claims 1 to 4, wherein the catalyst is maintained in its activated state by continuous contact with said olefin.

6. The process of any of Claims 1 to 4, wherein the catalyst is maintained in its activated state by continuous contact with said olefin being oxidized.

7. The process of any of Claims 1 to 6, wherein the catalyst is activated with said olefin under pressures of from about 1 to 100 atmospheres of said olefin.

8. The process of any of Claims 1 to 7, wherein the catalyst is activated with said olefin at temperatures of from about 60°C to 150°C for at least about 10 to 120 minutes.

9. The process of any of Claims 1 to 8, wherein the olefin is propylene and the carboxylic acid is acrylic acid.

10. The process of any of Claims 1 to 8, wherein the olefin is isobutylene and the carboxylic acid is methacrylic acid.

11. The process of any of Claims 1 to 8, wherein the olefin is butene-1 and the carboxylic acid is crotonic acid.

12. The process of any of Claims 1 to 11, wherein the support for the palladium metal is carbon or alumina.

13. The process of any of Claims 1 to 8, wherein the oxidation is carried out with stoichiometric amounts of propylene and oxygen needed to produce said carboxylic acid.

14. The process of any of Claims 1 to 13, wherein the oxidation is carried out at temperatures of at least about 25°C.

15. A process for preparing an activated palladium metal catalyst which comprises contacting a supported palladium metal catalyst in a liquid medium with a $C_3$—$C_6$ olefin at temperatures of at least about 60°C for at least about 10 minutes in the substantial absence of oxygen.

16. The process of Claim 15 wherein the catalyst is activated in the essential absence of oxygen.

17. The process of Claim 15 or 16, wherein the catalyst is prepared at temperatures of from about 60°C to 150°C for at least about 10 to 120 minutes.

18. The process of any of Claims 1 to 16, wherein the catalyst is prepared under about 1 to 100 atmospheres of olefin.

19. An olefin-activated palladium catalyst prepared by any of the processes defined in claims 15 to 18.

20. A process for the preparation of α,β-unsaturated carboxylic acids which comprises oxidizing a $C_3$—$C_6$ olefin with air or oxygen in an aqueous medium in the presence of an olefin—activated palladium catalyst as defined in Claim 19.

21. The process of Claim 20 wherein the olefin is propylene and the carboxylic acid is acrylic acid.

22. The process of Claim 20 wherein the olefin is isobutylene and the carboxylic acid is methacrylic acid.

23. The process of Claim 20 wherein the olefin is butene-1 and the carboxylic acid is crotonic acid.

24. The process of any of Claims 20 to 23, wherein the catalyst is maintained in its activated state by continuous contact with said olefin.

25. The process of any of Claims 20 to 24, wherein the oxidation is carried out at temperatures of at least about 25°C.

26. The process of any of Claims 20 to 25, wherein the oxidation is carried out with stoichiometric amounts of oxygen and olefin needed to produce said carboxylic acid.

**Patentansprüche**

1. Verfahren zur Herstellung α,β-ungesättigter Carbonsäuren, das folgende Verfahrensschritte umfaßt:

In-Kontakt-Bringen eines auf einem Träger aufgezogenen Palladiummetall-Katalysators mit einem $C_3$-bis $C_6$-Olefin in Abmischung mit Luft oder Sauerstoff und dadurch

Oxidieren des Olefins sur entsprechenden Carbonsäure, dadurch gekennzeichnet, daß

man den Katalysator zuerst dadurch aktiviert, daß man ihn mit dem $C_3$- bis $C_6$-Olefin in einem wäßrigen Medium bei einer Temperatur von wenigstens etwa 60°C für eine Zeit von wenigstens etwa 10 Minuten bei im wesentlichen vollständiger Abwesenheit von Sauerstoff in Kontakt bringt.

2. Verfahren zur Herstellung α,β-ungesättigter Carbonsäuren, das folgende Verfahrensschritte umfaßt:

In-Kontakt-Bringen eines auf einem Träger aufgezogenen Palladiummetall-Katalysators mit einem $C_3$-bis $C_6$-Olefin in Abmischung mit Luft oder Sauerstoff und dadurch

Oxidieren des Olefins zur entsprechenden Carbonsäure, dadurch gekennzeichnet, daß

man den Katalysator zuerst dadurch aktiviert, daß man ihn mit einem von dem genannten Olefin verschiedenen $C_3$- bis $C_6$-Olefin in einem wäßrigen Medium bei einer Temperatur von wenigstens etwa 60°C für eine Zeit von wenigstens etwa 10 Minuten bei im wesentlichen vollständiger Abwesenheit von Sauerstoff in Kontakt bringt.

3. Verfahren zur Herstellung α,β-ungesättigter Carbonsäuren, das den Verfahrensschritt der Oxidation eines $C_3$- bis $C_6$-Olefins in Abmischung mit Luft oder Sauerstoff in Gegenwart eines Palladiummetall-Katalysators in einem flüssigen Medium umfaßt, dadurch gekennzeichnet, daß der Kataysators dadurch aktiviert wurde, daß man einen auf einem Träger aufgezogenen Palladiummetall-Katalysator in dem flüssigen Medium mit einem von dem genannten Olefin verschiedenen $C_3$- bis $C_6$-Olefin bei einer Temperatur von wenigstens etwa 60°C für eine Zeit von wenigstens etwa 10 Minuten bei im wesentlichen vollständiger Abweseneheit von Sauerstoff in Kontakt bringt.

4. Verfahren nach einem der Ansprüche 1 bis 3, worin der Katalysator bei vollständiger Abwesenheit von Sauerstoff aktiviert wird.

5. Verfahren nach einem der Ansprüche 1 bis 4, worin der Katalysator durch andauernden Kontakt mit dem Olefin in aktiviertem Zustand gehalten wird.

6. Verfahren nach einem der Ansprüche 1 bis 4, worin der Katalysator durch andauernden Kontakt mit dem Olefin in oxidierter Form in aktiviertem Zustand gehalten wird.

7. Verfahren nach einem der Ansprüche 1 bis 6, worin der Katalysator mit dem Olefin unter Olefin-Drücken im Bereich von etwa 1 bis 100 Atmosphären aktiviert wird.

8. Verfahren nach einem der Ansprüche 1 bis 7, worin der Katalysator mit dem Olefin bei Temperaturen im Bereich von etwa 60°C bis 150°C für eine Zeit von wenigstens etwa 10 bis 120 Minuten aktiviert wird.

9. Verfahren nach einem der Ansprüche 1 bis 8, worin das Olefin Propylen ist und die Carbonsäure Acrylsäure ist.

10. Verfahren nach einem der Ansprüche 1 bis 8, worin das Olefin Isobutylen ist und die Carbonsäure Methacrylsäure ist.

11. Verfahren nach einem der Ansprüche 1 bis 8, worin das Olefin Buten-1 ist und die Carbonsäure Crotonsäure ist.

12. Verfahren nach einem der Ansprüche 1 bis 11, worin der Träger für das Palladiummetall Kohlenstoff oder Aluminiumoxid ist.

13. Verfahren nach einem der Ansprüche 1 bis 8, wocun die Oxidation mit stöchiometrischen Mengen Propylen und Sauerstoff durchgeführt wird, die zur Herstellung der Carbonsäure erforderlich sind.

14. Verfahren nach einem der Ansprüche 1 bis 13, worin die Oxidation bei einer Temperatur von wenigstens etwa 25°C durchgeführt wird.

15. Verfahren zur Herstellung eines aktivierten Palladiummetall-Katalysators, das den

## EP 0 145 467 B1

Verfahrensschritt des In-Kontakt-Bringens eines auf einem Träger aufgezogenen Palladiummetall-Katalysators mit einem $C_3$- bis $C_6$-Olefin in einem flüssigen Medium bei Temperaturen von wenigstens etwa 60°C über eine Zeit von wenigstens etwa 10 Minuten bei im wesentlichen vollständiger Abwesenheit von Sauerstoff umfaßt.

16. Verfahren nach Anspruch 15, worin der Katalysator bei vollständiger Abwesenheit von Sauerstoff aktiviert wird.

17. Verfahren nach Anspruch 15 oder 16, worin der Katalysator bei Temperaturen im Bereich von etwa 60°C bis 150°C über eine Zeit von wenigstens etwa 10 bis 120 Minuten hergestellt wird.

18. Verfahren nach einem der Ansprüche 1 bis 16, worin der Katalysator unter einem Olefin-Druck von etwa 1 bis 100 Atmosphären hergestellt wird.

19. Olefin-aktivierter Palladium-Katalysator, hergestellt nach einem der Verfahren nach Ansprüchen 15 bis 18.

20. Verfahren zur Herstellung von α,β-ungesättigten Carbonsäuren, das den Verfahrensschritt der Oxidation eines $C_3$- bis $C_6$-Olefins mit Luft oder Sauerstoff in einem wäßrigen Medium in Gegenwart eines Olefin-aktivierten Palladium-Katalysators nach Anspruch 19 umfaßt.

21. Verfahren nach Anspruch 20, worin das Olefin Propylen ist und die Carbonsäure Acrylsäure ist.

22. Verfahren nach Anspruch 20, worin das Olefin Isobutylen ist und die Carbonsäure Methacrylsäure ist.

23. Verfahren nach Anspruch 20, worin das Olefin Buten-1 ist und die Carbonsäure Crotonsäure ist.

24. Verfahren nach einem der Ansprüche 20 bis 23, worin der Katalysator durch andauernden Kontakt mit der Olefin in aktiviertem Zustand gehalten wird.

25. Verfahren nach einem der Ansprüche 20 bis 24, worin die Oxidation bei Temperaturen von wenigstens etwa 25°C durchgeführt wird.

26. Verfahren nach einem der Ansprüche 20 bis 25, worin die Oxidation mit stöchiometrischen Mengen Sauerstoff und Olefin durchgeführt wird, die zur Herstellung der Carbonsäure erforderlich sind.

**Revendications**

1. Procédé pour la préparation d'acides carboxyliques α,β-insaturés, qui comprend la mise en contact d'un catalyseur au palladium métallique sur support avec une oléfine en $C_3$ à $C_6$ mélangée avec de l'air ou de l'oxygène, afin d'oxyder cette oléfine en acide carboxylique correspondant, caractérisé en ce que le catalyseur est d'abord activé par mise en contact dans un milieu aqueux avec cette oléfine en $C_3$ à $C_6$, à une température au moins de l'ordre de 60°C pendant au moins environ 10 minutes, pratiquement en l'absence d'oxygène.

2. Procédé de préparation d'acides carboxyliques α,β-insaturés qui comprend la mise en contact d'un catalyseur au palladium métallique sur support avec une oléfine en $C_3$ à $C_6$ mélangée avec de l'air ou de l'oxygène, afin d'oxyder cette oléfine en acide carboxylique correspondant, caractérisé en ce que le catalyseur est d'abord activé par mise en contact dans un milieu aqueux avec une oléfine en $C_3$ à $C_6$ différente, à une température au moins de l'ordre de 60°C pendant au moins 10 minutes, pratiquement en l'absence d'oxygène.

3. Procédé de préparation d'acides carboxyliques α,β-insaturés qui comprend l'oxydation d'une oléfine en $C_3$ à $C_6$ en mélange avec de l'air ou de l'oxygène, en présence d'un catalyseur au palladium métallique dans un milieu liquide, caractérisé en ce que ce catalyseur a été activé par mise en contact d'un catalyseur au palladium métallique sur support dans ce milieu liquide avec une oléfine en $C_3$ à $C_6$ différente, à une température au moins de l'ordre de 60°C pendant environ au moins 10 minutes, pratiquement en l'absence d'oxygène.

4. Procédé selon une des revendication 1 à 3, dans lequel le catalyseur est activé essentiellement en l'absence d'oxygène.

5. Procédé selon une des revendications 1 à 4, dans lequel le catalyseur est maintenu en son état activé par contact continu avec cette oléfine.

6. Procédé selon une des revendications 1 à 4, dans lequel le catalyseur est maintenu en son état activé par contact continu avec cette oléfine à oxyder.

7. Procédé selon une des revendications 1 à 6, dans lequel le catalyseur est activé avec cette oléfine sous des pressions de cette oléfine allant de 1 à 100 atmosphères.

8. Procédé selon une des revendications 1 à 7, dans lequel le catalyseur est activé avec cette oléfine à des températures de l'ordre de 60° à 150°C pendant au moins environ 10 à 120 minutes.

9. Procédé selon une des revendications 1 à 8, dans lequel l'oléfine est de propylène et l'acide carboxylique est l'acide acrylique.

10. Procédé selon une des revendications 1 à 8, dans lequel l'oléfine est de l'isobutylène et l'acide carboxylique est l'acide méthacrylique.

11. Procédé selon une des revendications 1 à 8, dans lequel l'oléfine est du butène-1 et l'acide carboxylique est l'acide crotonique.

12. Procédé selon une des revendications 1 à 11, dans lequel le support du palladium métallique est du carbone ou de l'alumine.

9

13. Procédé selon une des revendications 1 à 8, dans lequel l'oxydation est réalisée avec les quantités stoechiométriques de propylène et d'oxygène nécessaire pour produire l'acide carboxylique.

14. Procédé selon une des revendications 1 à 13, dans lequel l'oxydation s'effectue à des températures de l'ordre d'au moins 25°C.

15. Procédé de préparation d'un catalyseur au palladium métallique activé qui comprend la mise en contact d'un catalyseur au palladium métallique sur support, dans un milieu liquide, avec une oléfine en $C_3$ à $C_6$, à des températures au moins de l'ordre de 60°C pendant au moins environ 10 minutes, pratiquement en l'absence d'oxygène.

16. Procédé selon la revendication 15, dans lequel le catalyseur est activé essentiellement en l'absence d'oxygène.

17. Procédé selon la revendication 15 ou 16, dans lequel le catalyseur est préparé à des températures de l'ordre de 60° à 150°C, pendant au moins environ 10 à 120 minutes.

18. Procédé selon une des revendications 1 à 16, dans lequel le catalyseur est préparé sous pression de l'oléfine de l'ordre de 1 à 100 atmosphères.

19. Catalyseur au palladium activé par oléfine, préparé conformément à l'un des procédé définis dans les revendications 15 à 18.

20. Procédé pour la préparation d'acides carboxyliques α,β-insaturés, qui compernd l'oxydation par l'air ou l'oxygène d'une oléfine en $C_3$ à $C_6$ dans un milieu aqueux, en présence d'un catalyseur au palladium activé par oléfine comme défini dans la revendication 19.

21. Procédé selon la revendication 20, dans lequel l'oléfine est du propylène et l'acide carboxylique est l'acide acrylique.

22. Procédé selon la revendication 20, dans lequel l'oléfine est de l'isobutylène et l'acide carboxylique est l'acide méthacrylique.

23. Procédé selon la revendication 20, dans lequel l'oléfine est du butène-1 et l'acide carboxylique l'acide crotonique.

24. Procédé selon une des revendications 20 à 23, dans lequel le catalyseur est maintenu à l'état activé par contact continu avec l'oléfine.

25. Procédé selon une des revendications 20 à 24, dans lequel l'oxydation est réalisée à des températures au moins de l'ordre de 25°C.

26. Procédé selon une des revendications 20 à 25, dans lequel l'oxydation s'effectue avec les quantités stoechiométriques l'oxygène et d'oléfine nécessaires pour produire l'acide carboxylique.